Europäisches Patentamt

European Patent Office

Office européen des brevets

(19)

(11) Veröffentlichungsnummer: **0 133 538**
**B1**

(12) **EUROPÄISCHE PATENTSCHRIFT**

(45) Veröffentlichungstag der Patentschrift: 14.03.90

(51) Int. Cl.⁵: **C 07 C 263/20**

(21) Anmeldenummer: **84109069.9**

(22) Anmeldetag: **01.08.84**

(54) **Verfahren zur Reinigung von Polyisocyanaten.**

(30) Priorität: **11.08.83 DE 3329124**

(43) Veröffentlichungstag der Anmeldung:
**27.02.85 Patentblatt 85/09**

(45) Bekanntmachung des Hinweises auf die
Patenterteilung:
**14.03.90 Patentblatt 90/11**

(84) Benannte Vertragsstaaten:
**BE DE FR GB IT NL**

(56) Entgegenhaltungen:
**DD-A- 127 328**
**DE-A-1 543 258**
**DE-A-2 532 722**
**GB-A- 887 874**
**GB-A-1 040 417**
**US-A-3 144 474**

(73) Patentinhaber: **BAYER AG**
**D-5090 Leverkusen 1 Bayerwerk (DE)**

(72) Erfinder: **Keggenhoff, Berthold, Dr.**
**Im Paradies 32**
**D-4150 Krefeld 29 (DE)**
Erfinder: **Richter, Franz-Moritz, Dr.**
**Holbeinweg 4**
**D-4047 Dormagen (DE)**
Erfinder: **Ellendt, Günther, Dr.**
**Deswatinesstrasse 81**
**D-4150 Krefeld (DE)**
Erfinder: **Petinaux, Marcel, Dr.**
**Flensburger Zeile 1h**
**D-4150 Krefeld (DE)**

Courier Press, Leamington Spa, England.

## Beschreibung

Die Erfindung betrifft ein neues Verfahren zur Reinigung von rohen Polyisocyanaten, insbesondere von rohen Polyisocyanatgemischen der Diphenylmethanreihe durch Extraktion bei erhöhter Temperatur und Gewinnung des gereinigten Polyisocyanats durch Abkühlung des Extrakts und anschließende Phasenscheidung, sowie die so gereinigten Polyisocyanate.

Es ist bereits in der Literatur beschrieben, daß rohe Isocyanatgemische durch Extraktion gereinigt werden können. So beschreibt die US—PS 3 144 474 ein Verfahren zur Reinigung von Isocyanaten, bei dem einer 30—60 %igen Lösung des Roh-Isocyanats in chlorierten aromatischen Kohlenwasserstoffen ein aliphatisches Kohlenwasserstoffgemisch zugesetzt wird, wodurch die Nebenprodukte ausgefällt werden. Das gereinigte Isocyanat muß durch Destillation aus dem Lösungsmittelgemisch gewonnen werden.

Die DE—OS 2 532 722 beschreibt weiterhin ein Verfahren zur Reinigung von 4,4'-Diphenylmethandiisocyanat (MDI). Hierbei wird ein Rohgemisch verwendet, das mindestens 80% 4,4'-MDI neben anderen Isomeren und bis 5 Gew.-% höhermolekulare Anteile enthält und üblicherweise durch Destillation aus dem durch Phosgenieren von Anilin-Formaldehyd-Kondensaten erhaltenen Isocyanatgemisch gewonnen wurde. Aus diesen werden durch Lösen in Kohlenwasserstoffen bei Temperaturen bis 80°C und Abkühlen Nebenprodukte und Isomere abgeschieden, worauf aus der Kohlenwasserstofflösung durch Rektifikation oder Kristallisation das 4,4'-MDI als Verfahrensprodukt gewonnen wird.

Schließlich beschreibt die DE—OS 1 543 258 ein Verfahren zur Reinigung von rohen Polyisocyanaten durch selektive Extraktion mit zwei Lösungsmitteln, die jeweils Löser und Nichtlöser für das Isocyanatgemisch darstellen, in bestimmten Mengenverhältnisse, wobei das gereinigte Polyisocyanat als Extrakt gewonnen wird und destillativ von den Extraktionsmitteln abgetrennt werden muß.

Die bekannten Verfahren haben des gemeinsame Merkmal, daß das gereinigte Isocyanat in einer, weger der erforderlichen Menge Extraktionsmitteln meist nur 5—20 %igen Lösung erhalten wird und zum Gewinnen des lösungsmittelfreien Isocyanats, das allein technisch verwendet wird, das Lösungsmittel mit hohem Energieaufwand abgestillt wird. Zudem ist diese destillative Aufarbeitung mit einer thermischen Belastung des Isocyanats verbunden, durch die gegebenenfalls neue Nebenprodukte erzeugt werden können.

Überraschenderweise wurde nun gefunden, daß diese Nachteile des Standes der Technik dadurch überwunden werden können, daß man die Extraktion von rohen Isocyanaten mit ausgewählten Lösungsmitteln bei erhöhter Temperatur durchführt und den Extrakt nach Abtrennung der Verunreinigungen abkühlt, wodurch das gereinigte Isocyanat sich als eigene Phase abscheidet und durch einfache Phasentrennung gewonnen werden kann, während die Lösungsmittelphase ohne weitere Reinigung erneut zur Extraktion eingesetzt wird. Obwohl somit mit undestilliertem Extraktionsmittel gearbeitet wird, wird überraschenderweise einer sehr gute Abtrennung von Nebenprodukten erzielt.

Gegenstand der Erfindung ist ein Verfahren zur Reinigung von rohen, durch Phosgenierung der ihnen zugrundeliegenden Polyamine erhaltenen Polyisocyanaten durch Extraktion, dadurch gekennzeichnet, daß man

a) das zu reinigende Polyisocyanat bei 80—180°C mit einem Extraktionsmittel unter Einhaltung eines Volumenverhältnisses von Extraktionsmittel zu Polyisocyanat von mindestens 5:1 extrahiert, wobei als Extraktionsmittel ein Lösungsmittel verwendet wird, welches gereinigtes Polyisocyanat der dem zu reinigenden Polyisocyanat entsprechenden Art gelöst enthält, und welches mit dem zu reinigenden Polyisocyanat innerhalb des genannten Temperaturbereichs nur begrenzt mischbar ist, so daß bei der Extraktion ein zweiphasiges System, bestehend aus einer Hauptphase und einer Nebenphase im Volumenverhältnis von mindestens 20:1 entsteht,

b) die aus der Hauptmenge des Lösungsmittels und gereinigtem Polyisocyanat bestehende Hauptphase von der, höhermolekulare Bestandteile und Verunreinigungen des zu reinigenden Polyisocyanats neben geringen Mengen an Lösungsmittel enthaltenden Nebenphase abtrennt,

c) die Hauptphase auf einer mindestens 50°C unter der Extraktionstemperatur liegende Temperatur unter Bildung eines zweiphasigen Systems abkühlt,

d) die hierbei anfallende, aus gereinigtem Polyisocyanat und Restmengen an Lösungsmittel bestehende, untere Phase von der, aus der Hauptmenge des mit gereinigtem Polyisocyanat gesättigten Lösungsmittel bestehenden, oberen Phase abtrennt und

e) die mit gereinigtem Polyisocyanat gesättigte Lösungsmittelphase, gegebenenfalls nach Zugabe von weiterem Lösungsmittel als Extraktionsmittel erneut zur Extraktion einsetzt.

f) und daß die zu reinigenden Polyisocyanate maximal 10 Gew.-% bezogen auf Gesamtgemisch, an chlorierten aromatischen Kohlenwasserstoffen enthalten

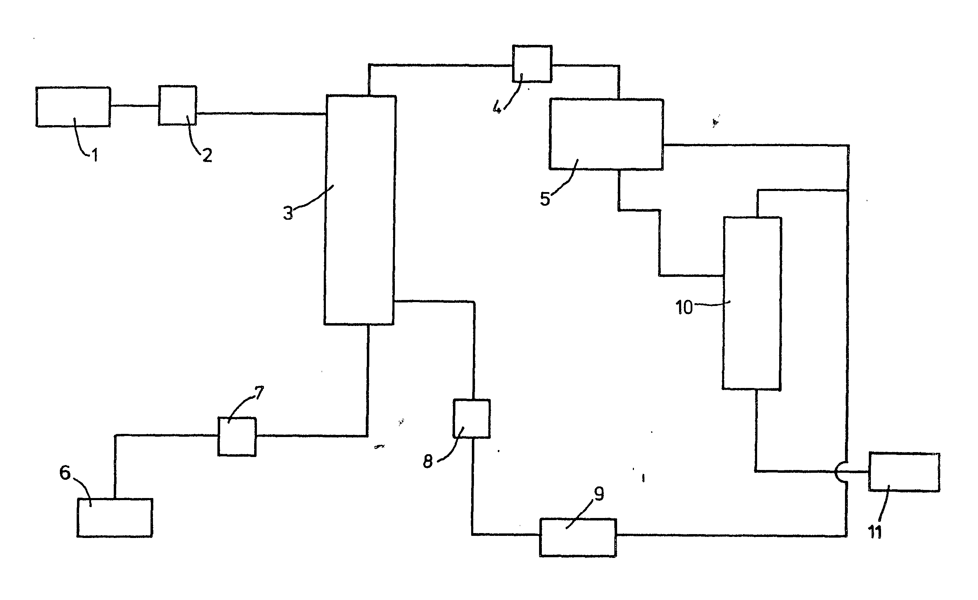

Das erfindungsgemäße Verfahren kann beispielsweise in der in der Zeichnung schematisch dargestellten Anlage durchgeführt werden. In dieser Zeichnung bedeuten im einzelnen

(1) einen Vorratsbehälter für zu reinigendes, rohes Polyisocyanat;

(2) einen Wärmeaustauscher (Erhitzer) zur Erhitzung des rohen Polyisocyanats auf die Extraktionstemperatur;

(3) eine Gegenstromextraktionskolonne;

(4) einen Wärmeaustauscher (Kühler) zur Kühlung der Lösung des gereinigten Polyisocyanats;

(5) einen Abscheider zur Trennung der Lösungsmittelphase von der Produktphase (gereinigtes Polyisocyanat);

(6) eine Vorlage für die angereicherte Verunreinigungen enthaltende Nebenphase;

(7) ein Wärmeaustauscher (Kühler) zur Abkühlung der den Extraktor verlassenden Nebenphase;

(8) einen Wärmeaustauscher (Erhitzer) für das in den Extraktor zu führende Extraktionsmittel;

(9) ein Lagertank für Extraktionsmittel;

(10) eine Destillationsstufe zur Abtrennung von Restlösungsmittel vom gereinigten Polyisocyanat und

(11) ein Lagertank für gereinigtes Polyisocyanat.

Die Tatsache, daß die in der Zeichnung schematisch dargestellte Anlage zur Durchführung des erfindungsgemäßen Verfahrens geeignet ist, soll nicht bedeuten, daß man bei der Durchführung des erfindungsgemäßen Verfahrens zwingend auf diese Anlage angewiesen ist. So kann auf an sich erfindungsunwesentliche Teilschritte ganz verzichtet werden, beispielsweise ist eine Abtrennung des Restlösungsmittel vom gereinigten Polyisocyanat in einer Destillationsstufe (10) nur dann erforderlich, wenn lösungsmittelfreies Polyisocyanat erhalten werden soll. Außerdem ist es selbstverständlich möglich, wesentliche Teilschritte des erfindungsgemäßen Verfahrens in anderen als den in der Zeichnung dargestellten Apparaturen durchzuführen. So kann beispielsweise die Extraktion a) und die anschließende Phasentrennung b) auch in einer Batterie von hintereinandergeschalteten Mischer-Scheider-Einheiten oder in mehreren hintereinandergeschalteten Extraktionskolonnen durchgeführt werden.

Ausgangsmaterialien für das erfindungsgemäße Verfahren sind rohe Polyisocyanate, wie sie bei der großtechnischen Phosgenierung der ihnen zugrundeliegenden Polyamine erhalten werden. Beispiele derartiger technischer Polyisocyanate sind Hexamethylendiisocyanat, 1-Isocyanato-3,3,5-trimethyl-5-isocyanatomethyl-cyclohexan, 2,4- und/oder 2,6-Diisocyanatotoluol und insbesondere Polyisocyanatgemische der Diphenylmethanreihe, wie sie durch großtechnische Phosgenierung von Anilin/Formaldehyd-Kondensaten erhalten werden, und die im wesentlichen Gemische aus 30 bis 90 Gew.-%, bezogen auf Gesamtgemisch, an Diisocyanato-diphenylmethan-Isomeren, insbesondere 4,4'-Diisocyanato-diphenylmethan mit höheren Homologen darstellen. Derartige Polyisocyanatgemische der Diphenylmethanreihe stellen die bevorzugten, nach dem erfindungsgemäßen Verfahren zu reinigenden, rohen Polyisocyanate dar. Im Unterschied zu den erstgenannten technischen Diisocyanaten können diese Polyisocyanatgemische nicht durch Destillation von höhermolekularen, harzartigen Bestandteilen, schwerflüchtigen chlor- oder eisenhaltigen Verbindungen und sonstigen, oft farbintensiven Verunreinigungen befreit werden, da im allgemeinen nur der Zweikern- und allenfalls der Dreikern-Anteil der Polyisocyanatgemische, nicht jedoch der höherkernige Anteil der Polyisocyanatgemische unzersetzt destillierbar ist. Man ist daher insbesondere bei den Polyisocyanatgemischen der Diphenylmethanreihe anstelle bzw. in Ergänzung der sonst üblichen destillativen Reinigungsmethoden auf andere Reinigungsverfahren angewiesen. Diesen Ausführungen entsprechend können anstelle der rohen Phosgenierungsprodukte von Anilin/Formaldehyd-Kondensaten beim erfindungsgemäßen Verfahren auch solche Polyisocyanatgemische der Diphenylmethanreihe eingesetzt werden, aus denen nach der Phosgenierung zunächst ein gewisse Menge an zweikernigen Diisocyanaten und gegebenenfalls dreikernigen Triisocyanaten abdestilliert worden sind, so daß lediglich die resultierenden Destillationsrückstände mit einem verminderten Anteil an Di- und gegebenenfalls Triisocyanat der erfindungsgemäßen Reinigungsoperation unterzogen werden.

Vor der Durchführung des erfindungsgemäßen Verfahrens muß darauf geachtet werden, daß die zum Einsatz gelangenden rohen Polyisocyanate einen Gehalt an starken Lösungsmitteln insbesondere solchen der bei der Phosgenierung üblicherweise mitverwendeten Art, d.h. insbesondere an Chlorbenzol bzw. Dichlorbenzol von max. 10 Gew.-%, bezogen auf Gesamtgemisch, aufweisen, da ein höherer Gehalt an derartigen Lösungsmitteln zur Folge hätte, daß die erfindungswesentliche, nachstehend näher beschriebene Mischungslücke zwischen Extraktionsmittel und zu reinigendem Polyisocyanat nicht mehr auftreten würde. Vorzugsweise weisen die zu reinigenden Polyisocyanate einen Gehalt an Lösungsmitteln der bei der Phosgenierung mitverwendeten Art von unter 0,5 Gew.-%, bezogen auf Gesamtgemisch, auf.

Das beim erfindungsgemäßen Verfahren eingesetzte Extraktionsmittel stellt eine Lösung von reinem Polyisocyanat der erfindungsgemäß herzustellenden Art in einem gegen- über Isocyanatgruppen inerten Lösungsmittel dar, welches bei der Extraktionstemperatur von 80—180°C, vorzugsweise 100 bis 150°C mit dem rohen Polyisocyanat nicht unbegrenzt mischbar ist. Im allgemeinen stellt das Extraktionsmittel eine bei der tiefsten Temperatur des Extraktionsmittelkreislaufes gesättigte oder nehezu gesättigte Lösung des reinen Polyisocyanats im Lösungsmittel dar, obwohl auch die Verwendung von reinem Lösungsmittel denkbar wäre, jedoch liefe eine Reindarstellung des im Kreislauf befindlichen Lösungsmittels, beispielsweise durch destillative Abtrennung des gelösten Polyisocyanats, lediglich auf eine an sich überflüssige Maßnahme hinaus. Die Forderung, daß das zum Einsatz gelangende Lösungsmittel mit dem zu reinigenden Polyisocyanat bei der Extraktionstemperatur nicht unbegrenzt mischbar sein darf, besagt, daß zwischen beiden Flüssigkeiten bei der Extraktionstemperatur eine Mischungslücke vorliegen muß, die zur Folge hat, daß bei der Extraktion bei Beachtung der sonstigen Parameter des Verfahrens, insbesondere des Mengenverhältnisses von Extraktionsmittel zu Polyisocyanat ein zweiphasiges, aus einer Haupt- und einer Nebenphase bestehendes System im Volumenverhältnis von mindestens 20:1 und bis zu mehr als 1000:1 entsteht.

Geeignete Lösungsmittel sind beispielsweise aliphatische Kohlenwasserstoffe, die unter Normaldruck oberhalb 90°C sieden, d.h. vorzugsweise unter Normaldruck einen Siedepunkt im Bereich von 95 bis 320°C oder einen beliebig breiten Siedebereich innerhalb dieses Temperaturbereichs aufweisen. Es handelt sich hierbei um gesättigte aliphatische Kohlenwasserstoffe einer Kettenlänge von 8 bis 18, vorzugsweise 8 bis 15 und besonders bevorzugt 10 bis 13 Kohlenstoffatomen oder um deren Isomeren- und/oder Homologengemische. Es können somit sowohl reine Kohlenwasserstoffverbindungen als auch deren technische Gemische verwendet werden, wobei sowohl verzweigte als auch geradkettige Paraffine oder beliebige Gemische derartiger Kohlenwasserstoffe in Betracht kommen. Das Lösungsmittel weist vorzugsweise einen Gehalt von mindestens 90 Gew.-% an derartigen aliphatischen Kohlenwasserstoffen auf, dies bedeutet insbesondere, daß der Gehalt des Lösungsmittels an aromatischen Kohlenwasserstoffen unter 10 Gew.-% liegen sollte.

Bei der Durchführung des vorzugsweise kontinuierlich betriebenen erfindungsgemäßen Verfahrens erfolgt in einem ersten Schritt die Extraktion des Ausgangspolyisocyanats, wobei die Menge des Extraktionsmittels in weiten Grenzen variiert werden kann. Im allgemeinen kommen mindestens 5 Vol.-Teile, vorzugsweise 5 bis 30 Vol.-Teile und besonders bevorzugt 10 bis 20 Vol.-Teile Extraktionsmittel pro Vol.-Teil Ausgangspolyisocyanat zum Einsatz. Die Extraktion erfolgt im allgemeinen unter Normaldruck, kann jedoch, wenn sich dies als zweckmäßig herausstellt oder aufgrund des Siedepunktes des Extraktionsmittels erforderlich wird auch unter Überdruck, beispielsweise bis zu 5 bar durchgeführt werden. Die Extraktionstemperatur liegt im Bereich von 80—180°C, vorzugsweise 100—150°C.

Für die Durchführung der Extraktion können prinzipiell alle einschlägig bekannten Vorrichtungen des Standes der Technik verwendet werden. Vorzugsweise erfolgt die Extraktion in Form einer Gegenstromextraktion, wobei z.B. Mischer-Scheider-Vorrichtungen oder Gegenstromextraktionskolonnen mit unbewegten oder bewegten Einbauten zum Einsatz kommen können.

Bei der Extraktion wird das rohe Polyisocyanat in dem erhitzten Lösungsmittel dispergiert und löst sich in ihm zum größten Teil unter Bildung der genannten "Hauptphase" auf. Die abzutrennenden Verunreinigungen und höhermolekularen Bestandteile scheiden sich mit geringen Lösungsmittelanteilen (bis zu 5 Gew.-% des eingesetzten Lösungsmittels) ab und bilden die genannte "Nebenphase". Die Menge der Nebenphase ist im wesentlichen durch die Extraktionstemperatur, das Phasenverhältnis und die Stufenzahl der Extraktion, aber auch durch den Nebenproduktgehalt des eingesetzten Isocyanats bestimmt und kann daher, wie oben dargelegt, in weiten Grenzen variiert werden. Da Ziel des Verfahrens aber die Abtrennung möglichst nur der Nebenprodukte ist, wird diese Menge durch Abstimmung dieser Parameter bevorzugt auf 1—25 Gew.-%, besonders bevorzugt 5—15 Gew.-% des Ausgangsprodukts (zu reinigendes Ausgangspolyisocyanat) eingestellt.

In einem zweiten Reaktionsschritt wird die Nebenphase von der Hauptphase abgetrennt und gegebenenfalls gesondert aufgearbeitet. Bei der bevorzugten Verfahrensvariante der Durchführung der Extraktion in Form einer Gegenstromextraktion erfolgt selbstverständlich der zweite Verfahrensschritt (Phasentrennung) gleichzeitig mit dem ersten Verfahrensschritt, so daß unmittelbar die Haupt- und Nebenphase als getrennte Phasen anfallen.

Die Hauptphase wird vorzugsweise in geeigneten Wärmeaustauschern, gegebenenfalls jedoch auch durch Verdampfungskühlung unter vermindertem Druck auf eine Temperatur abgekühlt, die mindestens 50°C, im allgemeinen 50—150°C unter der Extraktionstemperatur liegt. Hierbei entsteht aus der bei der erhöhten Extraktionstemperatur homogenen Hauptphase ein zweiphasiges Gemisch, welches in einem Phasenabscheider getrennt wird. Bei diesen Phasen handelt es sich einerseits um eine gesättigte Lösung von gereinigtem Polyisocyanat in der Hauptmenge des in der ursprünglichen "Hauptphase" vorgelegenen Lösungsmittels und andererseits um gereinigtes Polyisocyanat, welches noch geringe Restmengen an Lösungsmittel (im allgemeinen ca. 1—20 Gew.-%, bezogen auf das Gemisch aus gereinigtem Polyisocyanat und restlichem Lösungsmittel) enthält. Dieser Lösungsmittelanteil wird im allgemeinen vom gereinigten Polyisocyanat destillativ abgetrennt. Dabei kann dieses Destillat neben dem Lösungsmittel auch einen gewissen Anteil an Diisocyanat enthalten. Es wird mit der zuletzt genannten Lösungsmittelphase, die im allgemeinen bei der zuletzt genannten Phasentrennung die Oberphase bildet, vereinigt. Diese gegebenenfalls mit dem Restlösungsmittel vereinigte Lösungsmittelphase wird nach eventueller Zwischenlagerung erneut auf Extraktionstemperatur erhitzt und als Extraktionsmittel an den Prozeßanfang zurückgeführt. Das vorzugsweise vom restlichen Lösungsmittel destillativ befreite gereinigte Polyisocyanat entspricht der eingesetzten Menge an rohem Polyisocyanat, vermindert um die mit der genannten "Nebenphase" abgeschiedenen Menge und beträgt demzufolge vorzugsweise 75 bis 99 Gew.-%, besonders bevorzugt 85 bis 95 Gew.-% der eingesetzten Menge an rohem Polyisocyanat.

Gemäß einer bevorzugten Variante erfolgt die Abkühlung der "Hauptphase" und die Erhitzung des an den Prozeßanfang zurückzuführenden Extraktionsmittels in einem Gegenstromwärmeaustauscher, so daß der ohnehin geringe Energieaufwand des erfindungsgemäßen Verfahrens weiter reduziert werden kann.

Falls das eingesetzte Ausgangspolyisocyanat Restmengen an Lösungsmittel der bei der Phosgenierung verwendeten Art, d.h. insbesondere an chlorierten aromatischen Kohlenwasserstoffen enthält, verbleiben diese teilweise in der "Hauptphase" und müssen aus dieser entfernt werden. Dies geschieht zweckmäßigerweise vor und/oder während der Abkühlung der "Hauptphase", beispielsweise durch deren Entspannung unter Vakuum, wobei Abkühlung der Hauptphase und Abdestillieren der chlorierten Aromaten in einem Schritt erfolgt.

Das erfindungsgemäße Verfahren gestattet insbesondere die Reinigung von technischen Polyisocyanatgemischen der Diphenylmethanreihe ("MDI-Polymer-Typen") der obengenannten Art. Die erfindungsgemäß gereinigten MDI-Polymer-Typen weisen bei praktisch unverändertem Diisocyanatgehalt und praktisch unveränderter mittlerer Funktionalität eine im vergleich zum rohen Ausgangspolyisocyanat deutlich erniedrigte Viskosität auf. Trennt man aus ihnen destillativ einen gewissen Anteil an Diisocyanat ab, so erhält man Produkte mit dem Ausgangsprodukt entsprechender Viskosität, jedoch wesentlich höher NCO-Funktionalität. Die Gehalte der erfindugsgemäß gereinigten Polyisocyanate an Chlor sind deutlich und die Spurengehalte an Eisen, die einen starken Einfluß auf die Reaktivität der Polyisocyanate haben können, drastisch reduziert.

Ein weiterer wesentlicher Vorteil der erfindungsgemäß gereinigten MDI-Polymer-Typen ist in deren wesentlich verbesserter Farbe zu sehen. Während die ungereinigten rohen Phosgenierungsprodukte schwarze Flüssigkeiten darstellen, sind die entsprechenden erfindungsgemäß gereinigten Polyisocyanatgemische durchscheinende Flüssigkeiten von heller gelblicher Farbe.

Die erfindungsgemäß gereinigten Polyisocyanate eignen sich zur Herstellung von Polyurethanschaumstoffen oder auch zur Herstellung von massiven Formteilen und, bedingt durch ihre geringe Eigenfarbe auch zur Herstellung von Klebstoffen und Beschichtungen auf Polyurethanbasis.

Die nachfolgenden Beispiele dienen zur näheren Erläuterung des erfindungsgemäßen Verfahrens, ohne dieses zu beschränken.

Beispiel 1 (vgl. Zeichnung)

In einer Versuchsapparatur wird aus einer Vorlage (1) für rohes, lösungsmittelfreies MDI-Polymer (Analysendaten siehe unten) kontinuierlich ein Strom von 1 kg/h des Ausgangsmaterials über den Erhitzer (2) in den oberen Teil einer Siebbodenextraktionskolonne (3) mit der Länge von 180 cm, einem inneren Durchmesser von 5 cm und einem Siebbodenabstand von 5 cm geleitet. In dem Erhitzer (2) wird das Ausgangsmaterial auf 105°C erhitzt Gleichzeitig wird aus der Vorlage (9) über den Erhitzer (8) ein Strom von 20 kg/h Extraktionsmittel in den unteren Teil der Extraktionskolonne geleitet. Das Extraktionsmittel wird im Erhitzer (8) ebenfalls auf 105°C erhitzt. Bei dem Extraktionsmittel handelt es sich um eine bei 22°C nahezu gesättigte Lösung von, dem Ausgangspolyisocyanat entsprechendem, gereinigtem Polyisocyanat in einem Lösungsmittel, welches aus einem technischen Gemisch von 10 bis 13 Kohlenstoffatome aufweisenden n-Paraffinen besteht, welches unter Normaldruck einen Siedebereich von 190 bis 250°C aufweist. In der Extraktionskolonne werden das Ausgangspolyisocyanat und das Extraktionsmittel im Gegenstrom gegeneinander geführt. Die hierbei anfallende "Hauptphase" (20,89 kg/h) wird im Kühler (4) abgekühlt und das hierdurch entstehende zweiphasige Gemisch im Scheider (5) getrennt. Die hierbei entstehende Unterphase (0,94 kg/h) besteht zu 95 Gew.-% aus gereinigtem Polyisocyanat und zu 5 Gew.-% aus Lösungsmittel und wird in die Destillationsstufe (10) geleitet, die bei 240°C und 10 mbar im Sumpf betrieben wird. Nach Abtrennung des Restlösungsmittel (0,05 kg/h) fällt das gereinigte Polyisocyanat in einer Menge von 0,89 kg/h als Sumpf der Destillationsstufe (10) an und wird in der Vorlage (11) gesammelt.

Das als Oberphase des Abscheiders (5) anfallende, bei 22°C mit gereinigtem Polyisocyanat gesättigte Lösungsmittel (19,95 kg/h) wird mit dem als Kopfprodukt der Destillationsstufe (10) anfallenden Lösungsmittel (0,05 kg/h) vereinigt und in die Vorlage für Extraktionsmittel (9) geleitet.

Die im Sumpf der Extraktionskolonne (3) anfallende "Nebenphase" (0,12 kg/h) besteht zu 92 Gew.-% aus abgetrennten Verunreinigungen und höhermolekularen Bestandteilen des Ausgangspolyisocyanats und zu 8 Gew.-% aus Lösungsmittel. Nach Abkühlen auf Raumtemperatur im Kühler (7) wird es in der Vorlage (6) gesammtelt.

Das Ausgangsprodukt und das erfindungsgemäß gereinigte Produkt können wie folgt charakterisiert werden:

|  | Ausgangsprodukt | gereinigtes Produkt |
|---|---|---|
| % Diisocyanat (gel-chromatographisch) | 41,2 | 41,3 |
| % NCO (Mol 42) | 31,25 | 31,7 |
| Viskosität (mPas) | 202 | 86 |
| Chlorgehalt (%) | 0,29 | 0,20 |
| Eisengehalt (ppm) | 11 | 0,5 |
| Extinktion einer 5 %igen Lösung im Chlorbenzol bei 430 nm | 1,19 | 0,168 |

## Patentansprüche

1. Verfahren zur Reinigung von rohen, durch Phosgenierung der ihnen zugrundeliegenden Polyamine erhaltenen Polyisocyanaten durch Extraktion, dadurch gekennzeichnet, daß man

a) das zu reinigende Polyisocyanat bei 80—180°C mit einem Extraktionsmittel unter Einhaltung eines Volumenverhältnisses von Extraktionsmittel zu Polyisocyanat von mindestens 5:1 extrahiert, wobei als Extraktionsmittel ein Lösungsmittel verwendet wird, welches gereinigtes Polyisocyanat der dem zu reinigenden Polyisocyanat entsprechenden Art gelöst enthält, und welches mit dem zu reinigenden Polyisocyanat innerhalb des genannten Temperaturbereichs nur begrenzt mischbar ist, so daß bei der Extraktion ein zweiphasiges System, bestehend aus einer Hauptphase und einer Nebenphase im Volumenverhältnis von mindestens 20:1 entsteht,

b) die, aus der Hauptmenge des Lösungsmittels und gereinigtem Polyisocyanat bestehende Hauptphase von der, höhermolekulare Bestandteile und Verunreinigungen des zu reinigenden Polyisocyanats neben geringen Mengen an Lösungsmittel enthaltenden Nebenphase abtrennt,

c) die Hauptphase auf eine mindestens 50°C unter der Extraktionstemperatur liegende Temperatur unter Bildung eines zweiphasigen System abkühlt,

d) die hierbei anfallende, aus gereinigtem Polyisocyanat und Restmengen an Lösungsmittel bestehende, untere Phase von der, aus der Hauptmenge des mit gereinigtem Polyisocyanat gesättigten Lösungsmittel bestehenden, oberen Phase abtrennt und

e) die mit gereinigtem Polyisocyanat gesättigte Lösungsmittelphase, gegebenenfalls nach Zugabe von weiterem Lösungsmittel als Extraktionsmittel erneut zur Extraktion einsetzt

f) und daß die zu reinigenden Polyisocyanate maximal 10-Gew.-%, bezogen auf Gesamtgemisch, an chlorierten aromatischen Kohlenwasserstoffen enthalten.

2. Verfahren gemäß Anspruch 1, dadurch gekennzeichnet, daß man das gemäß d) erhaltene gereinigte Polyisocyanat destillativ von den Restmengen des Lösungsmittels befreit und das so erhaltene, gegebenenfalls isocyanathaltige Lösungsmittel mit dem an den Prozeßanfang zurückzuführenden, mit gereinigtem Polyisocyanat gesättigtem Lösungsmittel vereinigt.

3. Verfahren gemäß Anspruch 1 und 2, dadurch gekennzeichnet, daß man als Lösungsmittel aliphatische Kohlenwasserstoffe verwendet.

4. Verfahren gemäß Anspruch 1 bis 3, dadurch gekennzeichnet, daß man als zu reinigende Polyisocyanate rohe Polyisocyanatgemische der Diphenylmethanreihe, wie sie durch Phosgenierung von Anilin/Formaldehyd-Kondensaten erhalten werden, verwendet, die gegebenenfalls bis maximal 10 Gew.-%, bezogen auf Gesamtgemisch, an chlorierten aromatischen Kohlenwasserstoffen enthalten.

5. Verfahren gemäß Anspruch 1 bis 4, dadurch gekennzeichnet, daß man die Extraktion a) und die Phasentrennung b) im einer kontinuierlich betriebenen Extraktionskolonne durchführt.

**Revendications**

1. Procédé pour la purification par extraction de polyisocyanates bruts obtenus par phosgénation des polyamines dont ils dérivent, caractérisé en ce que:

a) on extrait le polyisocyanate à purifier à 80—180°C avec un agent d'extraction, tandis que l'on maintient un rapport en volume de l'agent d'extraction au polyisocyanate d'au moins 5:1, en utilisant comme agent d'extraction un solvant qui contient à l'état dissous le polyisocyanate purifié du type correspondant au polyisocyanate à purifier, et qui n'a qu'une miscibilité limitée avec le polyisocyanate à purifier dans l'intervalle de températures mentionné, de sorte qu'il se forme dans l'extraction un système à deux phases consistant en une phase principale et une phase secondaire dans un rapport en volume d'au moins 20:1;

b) on sépare la phase principale, consistant en la majeure partie du solvant et le polyisocyanate purifié, de la phase secondaire contenant des constituants de haut poids moléculaire et des impuretés du polyisocyanate à purifier, outre de faibles quantités de solvant;

c) on refroidit la phase principale à une température inférieure d'au moins 50°C à la température d'extraction, avec formation d'un système à deux phases;

d) on sépare la phase inférieure ainsi formée, consistant en polyisocyanate purifié et résidus de solvant, de la phase supérieure, consistant en la majeure partie du solvant saturé par le polyisocyanate purifié; et

e) on utilise à nouveau comme agent d'extraction pour l'extraction la phase de solvant saturée par le polyisocyanate purifié, éventuellement après addition de nouveau solvant; et en ce que

f) les polyisocyanates à purifier contiennent au maximum 10% en poids, par rapport au mélange total, d'hydrocarbures aromatiques chlorés.

2. Procédé selon la revendication 1, caractérisé en ce que l'on débarrasse le polyisocyanate purifié obtenu selon d) des résidus de solvant par distillation et on réunit le solvant contenant éventuellement l'isocyanate, ainsi obtenu avec le solvant saturé de polyisocyanate purifié à renvoyer au début du procédé.

3. Procédé selon les revendications 1 et 2, caractérisé en ce que l'on utilise comme solvants des hydrocarbures aliphatiques.

4. Procédé selon les revendications 1 à 3, caractérisé en ce que l'on utilise comme polyisocyanates à purifier des mélanges bruts de polyisocyanates de la série du diphénylméthane, tels qu'on les obtient par phosgénation de condensats aniline-formaldéhyde, qui contiennent éventuellement jusqu'à 10% en poids au maximum d'hydrocarbures aromatiqués chlorés, par rapport au mélange total.

5. Procédé selon les revendications 1 à 4, caractérisé en ce que l'on met en oeuvre l'extraction a) et la séparation de phases b) dans une colonne d'extraction fonctionnant en continu.

**Claims**

1. Process for purifying crude polyisocyanates obtained by phosgenating the polyamines on which they are based by extraction, characterised in that

a) the polyisocyanate to be purified is extracted using an extraction agent at 80—180°C while maintaining a volume ratio of extraction agent to polyisocyanate of at least 5:1, wherein a solvent which contains dissolved purified polyisocyanate of the type appropriate to the polyisocyanate to be purified, and which can be mixed only to a limited extent within the temperature range mentioned with the polyisocyanate to be purified, is used as extraction agent, so that the volume ratio is at least 20:1 when extracting a two-phase system comprising one main phase and one secondary phase,

b) the main phase comprising the main amount of solvent and purified polyisocyanate is separated from the secondary phase containing higher molecular components and impurities of the polyisocyanate to be purified in addition to small amounts of solvent,

c) the main phase is cooled to a temperature at least 50°C below the extraction temperature while forming a two-phase system,

d) the lower phase resulting from the process comprising purified polyisocyanate and residual amounts of solvent is separated from the upper phase comprising the main amount of the solvent saturated with purified polyisocyanate, and

e) the solvent phase saturated with purified polyisocyanate optionally after adding further solvent, is used again for extraction, as extraction agent.

f) and in the polyisocyanates to be purified contain a maximum of 10 wt.%, relative to the total mixture, of chlorinated aromatic hydrocarbons.

2. Process according to claim 1, characterised in that the purified polyisocyanate obtained in accordance with d) is separated from the residual amounts of solvent by distillation, and the solvent thus obtained, optionally containing isocyanate, is combined with the solvent saturated with purified polyisocyanate to be recycled to the start of the process.

3. Process according to claim 1 and 2, characterised in that aliphatic hydrocarbons are used as solvent.

4. Process according to claim 1 to 3, characterised in that crude polyisocyanate mixture of the diphenylmethane series, as obtained by phosgenation of aniline/formaldehyde condensates, and which optionally contain up to a maximum 10 wt.%, relative to total mixture, of chlorinated aromatic hydrocarbons, are used as polyisocyanates to be purified.

5. Process according to claim 1 to 4, characterised in that extraction a) and phase separation b) are carried out in a continuously operated extraction column.